# NOUVEAU FASCICULE DE BREVET EUROPEEN

(11) **EP 0 418 153 B2**
(45) Date de publication et mention de la décision concernant l'opposition: **27.03.1996**
(45) Mention de la délivrance du brevet: 25.08.1993
(21) Numéro de dépôt: 90402509.5
(22) Date de dépôt: 12.09.1990
(51) Int. Cl.: A61K 9/16, A61K 9/127

(54) **Nouveau procédé de préparation de microparticules lipidiques**
Verfahren zur Herstellung von lipidischen Mikropartikeln
Preparation of lipidic microparticles

(30) Priorité: 14.09.1989 FR 8912038
(43) Date de publication de la demande: 20.03.1991
(73) Titulaire: THE LIPOSOME COMPANY, INC., Princeton, NJ 08540 (US)
(72) Inventeur: Leclef, Brigitte, B-1200 Bruxelles (BE); Cerfontaine, Patrick, B-1090 Bruxelles (BE); Nicolas, Jean-Marie, B-1900 Overijse (BE); Wantier, Henri, B-7270 Dour (BE); Trouet, André, B-3009 Winksele Herent (BE)
(74) Mandataire: Warcoin, Jacques

(56) Documents cités:
- EP-A- 0 253 619
- EP-A- 0 270 460
- EP-A- 0 349 429
- WO-A-90/11780
- US-A- 4 830 858
- Derwent-Ref. à SU-A-1186212
- S. Batzri et E. Korn "Single bilayer liposomes prepared without sonication", Biochimica et Biophysica Acta 298 (1973), pp. 1015-1019
- Römpps Chemie-Lexikon, 8ème édition (1983), pp. 2380 et 4508
- G. Gregoriadis, Liposome Technology, vol. 1, CRC Press Inc., Boca Raton, Florida (1984), p. 251.

## Description

La présente invention concerne un nouveau procédé de préparation de microparticules lipidiques d'aspect microcristallin.

Par "aspect cristallin" on n'entend pas que la structure cristalline soit nécessairement obtenue stricto sensus.

Ce type de microparticules a été décrit par la Demanderesse notamment dans la demande de brevet européen n° 270 460.

Plus précisément, il s'agit de microparticules d'une substance insoluble dans l'eau présentant une affinité pour les phospholipides et d'au moins un phospholipide.

Il faut entendre par "substance insoluble dans l'eau" une substance peu ou pas soluble dans l'eau et par "substance présentant une affinité pour les phospholipides" un composé chimique plus particulièrement capable d'interagir avec les phospholipides par voie physico-chimique.

Ces microparticules offrent principalement l'avantage d'obtenir une microsuspension stable en solution aqueuse d'une substance par ailleurs insoluble en phase aqueuse, ce qui permet de l'administrer notamment lorsqu'il s'agit d'un médicament sous forme injectable ou pulvérisable.

En outre, vraisemblablement en vertu d'un effet de pilotage accrû notamment vers les macrophages d'une part, et d'un effet de libération progressif de la substance active d'autre part, des préparations de microparticules donnent des résultats d'activité thérapeutique et de toxicité nettement plus intéressants que les vésicules lipidiques de type liposome.

On a décrit dans la demande EP 270 460 un procédé de préparation relativement complexe de ces microparticules inspiré des procédés utilisés pour la préparation des liposomes.

Le procédé décrit dans EP 270 460 comporte essentiellement les étapes suivantes :
a) on évapore les solvants d'une solution de phospholipide dans le chloroforme et de ladite substance dans le méthanol,
b) on remet le film obtenu après évaporation desdits solvants en suspension dans une solution aqueuse après agitation vigoureuse.

Pour remettre le film obtenu à l'étape b) en suspension, ceci était effectué par traitement aux ultrasons.

Dans le cadre d'une application industrielle du procédé, la complexité de celui-ci, avec notamment la présence d'une étape d'agitation vigoureuse et notamment de traitement aux ultrasons, constitue un inconvénient majeur.

La Demanderesse a maintenant découvert que ces microparticules pouvaient être préparées par un procédé beaucoup plus simple et avec un bien meilleur rendement.

En particulier, selon le procédé objet de la présente invention. l'étape de sonication est supprimée et l'on n'utilise plus de chloroforme pour solubiliser le phospholipide.

En effet, la présente invention a pour objet un procédé de préparation de microparticules lipidiques d'aspect microcristallin d'une substance insoluble dans l'eau présentant une affinité pour les phospholipides, et d'au moins un phospholipide, microparticules stables en suspension dans une solution aqueuse, caractérisé en ce que :
a) on dissout ladite substance et le ou lesdits phospholipides dans un solvant organique commun de ladite substance et du ou desdits phospholipides,
b) on mélange la solution obtenue de ladite substance et du ou desdits phospholipides à une solution aqueuse dans une quantité telle qu'on observe une insolubilisation sous forme de précipité,
c) on élimine le solvant organique pour récupérer une solution aqueuse contenant les microparticules sous forme de microsuspensions.

L'élimination du solvant organique peut se faire par évaporation, centrifugation ou ultrafiltration.

La simplicité du procédé selon la présente invention provient du fait que des complexes substance-phospholipides sont vraisemblablement formés dès la mise en présence des différents constituants résultant d'une interaction physico-chimique. L'apport de la solution aqueuse entraine la précipitation sous forme de microparticules desdits complexes.

Selon la présente invention, on obtient des microparticules d'aspect, de structure et de taille similaires et des préparations de microparticules plus homogènes présentant même des propriétés pharmacologiques avantageuses par rapport à des lots obtenus par le procédé précédemment décrit dans EP 270 460.

Comparé à la préparation de liposomes et à la préparation des microparticules selon le procédé antérieur, le procédé selon la présente invention est beaucoup plus simple et plus économique. On obtient en particulier beaucoup moins de substances libres dans la solution finale et donc un meilleur rendement et les préparations de microparticules obtenues ont une plus grande pureté que celles obtenues par le précédent procédé. Elles sont essentiellement dépourvues d'amphotéricine B libre ou de phospholipide libre. En outre, les microparticules obtenues sont aussi stables que celles obtenues par l'ancien procédé et donc beaucoup plus stables que les liposomes qui souffrent d'une instabilité qui en limite considérablement les possibilités d'emploi. L'avantage du procédé selon l'invention est en effet ainsi notable en termes de reproductibilité et d'homogénéité des lots de multiparticules produits. La présente invention a donc également pour objet des préparations de microparticules obtenus par le procédé selon l'invention caractérisés en ce qu'elles sont essentiellement dépourvues en substance active libre et en phospholipide libre et que les microparticules sont de tailles homogènes.

A titre de solvants organiques utiles dans le procédé selon l'invention, on peut citer plus particulièrement les solvants de polarité intermédiaire tels que le méthanol, le diméthylformamide (DMF), le diméthylacétamide (DMA), ou le propylène glycol ou l'éthanol.

Comme solution aqueuse utile dans le procédé selon la présente invention, on utilisera avantageusement l'eau pure ou un tampon phosphate ou des solutions salines telles qu'une solution de NaCl, par exemple à concentration de 0,5 à 1 %, par exemple 0,9 % (poids/volume) ou une solution de saccharose de 1 à 10 % (poids/volume), par exemple du lactose ou du glucose sont particulièrement appropriés en vue d'une lyophilisation ultérieure.

On cite en particulier une solution tampon phosphate 50mM pH=7,8 contenant 6% de lactose qui permet d'obtenir des préparations très homogènes en taille.

Selon la présente invention, le rapport molaire phospholipide(s)/substance engagés dans le procédé peut être compris entre 0.1 et 10.

Comme on l'a vu, les microparticules selon l'invention résultent vraisemblablement d'une complexation substance-phospholipide dans un rapport molaire voisin de 1/1. C'est pourquoi, si le rapport molaire est inférieur ou égal à 2, c'est-à-dire par exemple entre 0,5 et 2, de préférence entre 0,5-1, on observe une interaction spécifique avec production homogène optimum de microparticules dont la taille moyenne est inférieure à 1 µm.

Dans les préparations de microparticules obtenues par le procédé selon l'invention, la taille des microparticules est homogène et se répartit en général entre 0,5 et 2 µm, certaines pouvant dépasser cette fourchette mais restant entre 0,1 et 10 µm.

Comme phospholipides utiles dans le procédé, on peut utiliser la phosphatidylcholine, la dimyristoylphosphatidylcholine (DMPC), la distéaryl phosphatidyl choline (DSPC), la dipalmitoylphosphatidylcholine (DPPC), la phosphatidyléthanolamine, la phosphatidylsérine, la dipalmitoyl phosphatidyl serine (DPPS), la phsophatidylinositol, la phosphatidylglycérol, la dimyristoyl phosphatidyl glycérol (DMPG), la distearyl phosphatidyl glycérol (DSPG), la 3'O-lysylphosphatidylglycérol, la diphosphatidyl glycérol, ou encore des esters de cholestérol, seuls ou en mélange, cette liste n'étant bien entendu pas limitative.

Selon l'invention, on utilisera de préférence comme phospholipide de la phosphatidylcholine ou dimyristoyl phosphatidylcholine en mélange avec du dimyristoyl phosphatidyl glycerol. La phosphatidylcholine peut être préparée à partir de lécithine de jaune d'oeuf, de soja hydrogénée ou non ou de tout autre source industrielle de lécithine.

Certaines substances présentent une plus grande solubilité en solution organique dans un solvant donné lorsqu'on se trouve en milieu acide ou basique et sous forme de sel et qu'elles sont ionisées.

Dans ce cas, on prépare avantageusement les microparticules selon l'invention de la manière suivante :
a) on dissout ladite substance et le ou lesdits phospholipides dans leur solvant organique commun en milieu basique ou acide,
b) on mélange la solution obtenue avec une solution aqueuse pour obtenir un précipité, et on neutralise la solution par addition d'acide ou de base respectivement, la neutralisation pouvant se faire avant ou après addition de la solution aqueuse.
c) on élimine le solvant pour récupérer une phase aqueuse contenant les microparticules sous forme de microsuspensions.

On peut également laver les microparticules à l'eau par des cycles répétés de centrifugation et d'élimination du surnageant afin d'éliminer le maximum de solvant organique.

A l'étape a) ,on introduire de façon appropriée de 1 à 1,5 équivalent de base ou d'acide le cas échéant par rapport à ladite substance, et on neutralisera par la même quantité d'acide ou de base respectivement.

On observe un début de précipitation à l'étape a).

Le procédé selon l'invention est particulièrement intéressant pour préparer des microparticules de médicaments antimycotiques macrolides polyènes, tels que la nistatine et l'amphotéricine B et leurs dérivés qui présentent également une activité antifongique.

Lorsque dans le procédé selon l'invention, ladite substance est l'amphotéricine B, les meilleurs résultats ont été obtenus en utilisant un mélange de phosphatidylcholine (lécithine d'oeuf ou lécithine de soya hydrogénée) ou DMPC et de DMPG en rapports molaires variables de 5:5 à 9:1.

Dans le cas où la substance active est un macrolide de type polyène tel que l'amphotéricine B ou la nystatine, lorsqu'elle est dissoute dans du méthanol ou du propylène glycol, la solution initiale de la substance et du phospholipide sera avantageusement une solution basique, par exemple une solution comportant de 1 à 1,5 équivalent de base tel que NaOH ou KOH par rapport à la substance active. On neutralise ultérieurement la solution par addition de 1 à 1,5 équivalent d'acide tel que HCl. L'équivalent représente en l'espèce autant de moles de base ou d'acide que de moles d'amphotéricine.

Dans le cas où la substance active est un macrolide de type polyène tel que l'amphotéricine B ou la nystatine et lorsqu'elle est dissoute dans une solution d'éthanol ou de DMF, avantageusement la solution initiale de la substance et du phospholide sera une solution acide, par exemple une solution comportant de 1 à 1,5 équivalent d'acide tel que HCL par rapport à la substance active. On neutralise ultérieurement la solution dans le procédé par addition de 1 à 1,5 équivalent de base tel que NaOH ou KOH.

Lorsque le solvant est le DMA, on peut également avoir recours à une solution acide bien que cela ne soit pas obligatoire, la solubilité des macrolides polyènes tels que l'amphotéricine B dans le DMA étant suffisante pour éviter l'utilisation d'un acide. Il demeure que la solubilité de l'amphotéricine B est améliorée en solution acide dans le DMA.

Dans certains cas, notamment lorsque l'élimination du solvant ne se fait pas par chauffage avantageusement de manière à obtenir des microparticules plus stables, on chauffe le mélange avant l'élimination du solvant à l'étape c), par exemple 30 minutes à au moins 40°C, de préférence 60°C.

De même pour éviter un risque de dégradation de la substance active en milieu acide ou basique, on pourra solubiliser en la refroidissant dans un bain de glace à l'étape a).

Le traitement des infections mycotiques causées par Candida et Aspergillus est difficile et habituellement mal toléré. Peu de médicaments sont actifs contre ces deux types de microorganismes.

Les antimycotiques macrolides polyènes tels que la nystatine et l'amphotéricine B sont les produits les plus utilisés et caractérisés par une activité tant sur l'espèce Aspergillus que l'espèce Candida.

L'utilisation clinique de l'amphotéricine B est très fortement limitée par deux inconvénients majeurs :
- en premier lieu, sa grande insolubilité qui nécessite son administration en solution dans du désoxycholate de sodium,
- en second lieu, la toxicité intrinsèque de désoxychlotate qui s'additionne à l'activité toxique de l'amphotéricine B proprement dite et qui s'exerce surtout au niveau des reins et de la moelle osseuse.

Cependant, quels que soient ses effets secondaires, cet antibiotique reste efficace dans les infections fongiques qui auraient un pronostic mortel sans ce traitement. Dans ce contexte, il est apparu important de diminuer la toxicité de l'amphotéricine B en modifiant sa pénétration intracellulaire.

En effet, un facteur très important pour l'efficacité des antimycotiques en général, et pour l'amphotéricine B en particulier, est la nécessité d'induire une action antibiotique qui soit synergique avec celle des cellules hôtes, responsables des défenses anti-infectieuses de l'organisme. Il a en effet été démontré que les polymorphonucléaires et les macrophages peuvent maîtriser avec succès, des infections mycotiques dans la mesure où ces cellules peuvent phagocyter les microoganismes et les contrôler dans leur système lysosomial. Des médicaments antimycotiques doivent dès lors, non seulement réduire la multiplication des agents infectieux présents dans le milieu extracellulaire mais pouvoir exercer également leur action à l'intérieur des phagosomes et lysosomes de polymorphonucléaires et macrophages. Or, très peu de choses sont connues de la pénétration intracellulaire des macrolides polyènes et tout laisse supposer par ailleurs que ces substances s'accumulent au niveau des membranes pericellulaires et ne parviennent dès lors à atteindre les compartiments lysosomiaux intracellulaires que grâce au flux membranaire de la surface vers les espaces intracellulaires.

L'invention a apporté une solution à ces problèmes en proposant une nouvelle forme galénique de l'amphotéricine B basée sur l'interaction de ce médicament avec la phosphatidylcholine pour former une suspension de microparticules évoquée ci-dessus.

Cette formule galénique permet en premier lieu d'administrer l'amphotéricine B sans avoir recours à du désoxycholate et elle permet de décroître très significativement la toxicité aiguë des produits après injection intraveineuse. Ces microparticules ont la même activité que l'amphotéricine solubilisée par du désoxycholate contre des Candida extracellulaires et sont plus actifs in vitro dans les infections intracellulaires à macrophages.

Les propriétés des microparticules d'amphotéricine B ont également été comparées avantageusement à celles des liposomes contenant le même macrolide polyène et qui sont également préconisées comme transporteur capable de réduire les effets secondaires de l'amphotéricine.

D'autres caractéristiques et avantages de la présente invention apparaîtront à la lumière de la description qui va suivre faisant référence à des figures.

Les figures 1 et 2 représentent les courbes de stabilité de l'amphotéricine B en milieu acide et basique respectivement, à diverses concentrations et à température ambiante.

Les figures 3 à 16 représentent les profils de densité des centrifugations en gradient de sucrose pour différentes fractions de préparation d'amphotéricine B selon les procédures 1 à 14 respectivement.

### EXEMPLE 1 : Microparticule d'amphotéricine B et de phosphatidylcholine

### 1. Solubilité de l'amphotéricine B

L'amphotéricine B est une composé amphotère présentant à la fois une partie de sa structure qui est polaire (avec des groupes acide et des groupes amine) et une partie non polaire. Excepté dans le DMSO et le DMF, l'amphotéricine B se dissout faiblement dans la plupart des solvants organiques. Toutefois, l'ionisation des groupes acide et amine augmente sa solubilité (voir tableau 1)

**Tableau 1**

| Solubilité de l'amphotéricine B dans différents solvants | | | |
|---|---|---|---|
| pH | H₂O | CH₃OH | Diméthylformamide |
| neutre | insoluble | 0,2-0,4 mg/ml | 2,0-4,0 mg/ml |
| acide | 0,1 mg/ml | 3,0-5,0 mg/ml | 60,0-80,0 mg/ml |
| basique | 0,1 mg/ml | 2,0-3,0 mg/ml | --------- |

Deux lots différents d'amphotéricine B ont été utilisés pour les tests décrits ci-après :
- amphotéricine B pour application topique,
- amphotéricine B pour injection i.v.

### 1.1 Solubilité de l'amphotéricine B "grade topique"

| Solvant | Solubilité (mg/ml) |
|---|---|
| CH₃OH | 0,13 |
| CH₃OH + 1,5 eq. HCl | 5,00* |
| CH₃OH + 1,5 eq.NaOH | 5,00 |

| | |
|---|---|
| * voir § 2.3 | |

### 1.2 Solubilité d'amphotéricine B "grade i.v."

| Solvant | Solubilité (mg/ml) |
|---|---|
| CH₃OH | 0,150 |
| CH₃OH + 1,0 eq. HCl | 5,00* |
| CH₃OH + 1,0 eq. NaOH | 5,00 |

| | |
|---|---|
| * dissolution voir § 2.3 | |

L'acide acétique et l'ammoniac NH₃ dans le méthanol ne dissolvent pas l'amphotéricine B (5 mg/ml dans CH₃OH) après addition de deux équivalents desdits réactifs.

### 2. Stabilité des solutions d'amphotéricine B

### 2.1 Méthode

La stabilité des solutions d'amphotéricine B a été enregistrée par analyse HPLC en utilisant une colonne à phase inverse (C18 Bondapak de 25 x 0,5 cm, Waters Associates). On a utilisé comme éluant un système CH₃CN/tampon acétate 10 mM, pH 7 (39:61). On a effectué l'enregistrement U.V. à 404 et 362 nm. Le débit était de 2 ml/minutes. Le temps de rétention (R.T.) de l'amphotéricine B est de 9,5 minutes. Des résultats ont été exprimés comme le pourcentage de la surface des pics.

### 2.2 Solutions de CH₃OH neutre

A une concentration de 0,1 mg/ml, l'amphotéricine B est stable pendant plusieurs jours à 4°C. L'amphotéricine B grade topique a une pureté de 88,5 % (contamination de 7,6 % avec un temps de rétention de 4,5 minutes).

L'amphotéricine B de grade i.v. a une pureté de 94,5 % (contamination de 3,5% avec un temps de rétention de 4,5 minutes).

### 2.3 Solutions de méthanol en milieu acide

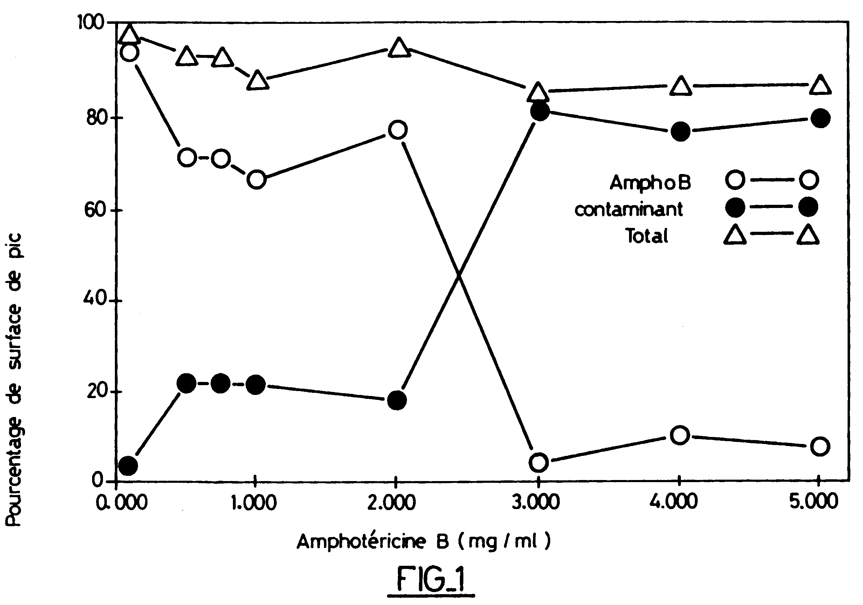

Les essais avec l'amphotéricine B grade topique ont montré que le composé est stable à une concentration de 0,1 mg/ml en présence de 1,5 eq. de HCl dans la solution méthanolique, mais se dégrade dans ce milieu acide à une concentration de 5 mg/ml (voir figure 1). Les résultats HPLC obtenus pour une solution d'amphotéricine B de 0,1 mg/ml sont reproduits dans le tableau 2 ci-après. La neutralisation a été effectuée par contrôle du pH. L'addition d'un excès de NaOH n'affecte pas la stabilité du composé (tableau 2).

**Tableau 2**

| Echantillon | pH | Amphotéricine B (%) R.T. : 9,5 minutes | Contamination (%) R.T.:4,5 minutes |
|---|---|---|---|
| Ampho. B 0,1 mg/ml | 7,1 | 89,1 | 7,6 |
| id. + 1,5 eq. HCl | 3,7 | 88,2 | 7,7 |
| id. + 1,5 eq. NaOH | 7,1 | 87,3 | 8,1 |
| id. + excès de NaOH | 7,5 | 87,3 | 8,1 |
| id. + excès de NaOH | 9,5 | 87,3 | 8,1 |

Afin d'étudier l'effet de l'addition de HCl sur la stabilité de l'amphotéricine B, des quantités croissantes d'amphotéricine B (grade i.v.) ont été dissoutes dans le méthanol en présence d'un équivalent de HCl (solution méthanolique). La dégradation en milieu acide de la substance de l'amphotéricine B augmente fortement quand la concentration d'amphotéricine B est supérieure à 2 mg/ml (voir figure 1) et ceci même quand l'addition de HCl en solution méthanolique a été faite à 0°C (avec une contamination de 31 %). Pour obtenir une solution d'amphotéricine B/HCl à une concentration de 5 mg/ml sans dégradation de l'amphotéricine B, l'amphotéricine B a été dissoute dans du méthanol en présence d'un équivalent de HCl à une concentration de 2 mg/ml. Le solvant a ensuite été évaporé et le sel d'acide de l'amphotéricine B a été redissous dans le volume requis d'alcool.

### 2.4 Solutions de CH₃OH basique

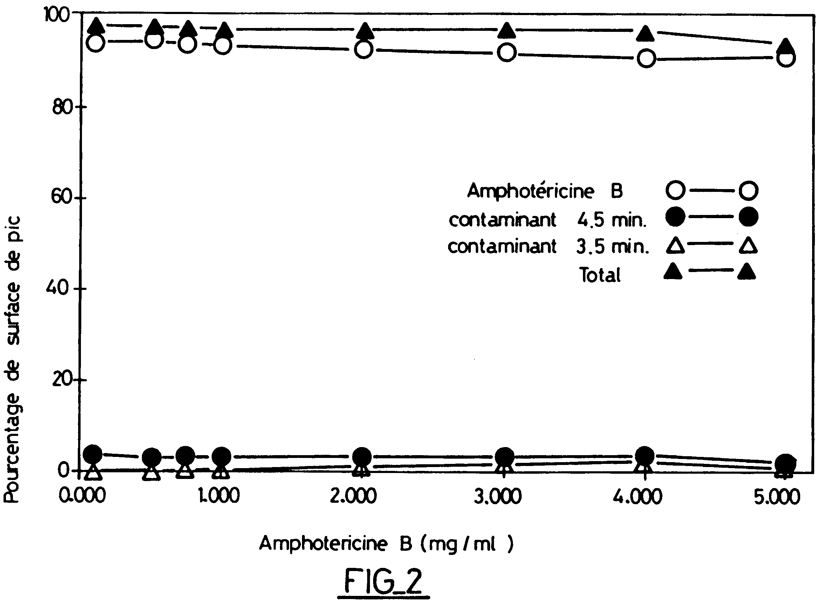

Des quantités croissantes d'amphotéricine B (grade i.v.) ont été dissoutes dans du méthanol en présence d'un équivalent de NaOH (solution méthanolique). Les solutions ont été ensuite analysées par HPLC. Comme il apparat sur la figure 2, l'amphotéricine B a une bonne stabilité en solution méthanolique de NaOH jusqu'à une concentration de 5 mg/ml. La neutralisation avec de l'HCl méthanolique n'a pas d'effet sur la stabilité du produit.

### 3. Dosage de l'amphotéricine B

Pour doser l'amphotéricine B par analyse U.V., la valeur **E** a été déterminée en mesurant l'absorbance à 404 nm pour des quantités croissante d'amphotéricine B dissoutes dans du DMF.

L'amphotéricine B (mg/ml) : (Abs./49,8) x facteur de dilution.

Des contrôles appropriés ont montré que les phospholipides et le sucrose n'interfèrent pas avec le dosage de l'amphotéricine B.

### 4. Préparation des microparticules

### 4.1 Introduction

Dans le but d'améliorer la préparation des microparticules, différents procédés ont été comparés. Plusieurs paramètres ont été évalués :
a) l'addition de NaOH ou HCl pour augmenter la solubilité d'amphotéricine B dans le méthanol,
b) l'effet de la dissolution du phospholipide dans du chloroforme avant l'addition de la solution méthanolique d'amphotéricine B,
c) l'effet de la formation d'un film de phospholipide préalablement à l'addition de la solution méthanolique d'amphotéricine B.

La centrifugation en gradient de densité a été choisie comme méthode analytique pour déterminer la qualité des microparticules.

### 4.2 Matériels et méthodes

### * Matériels

La L-alpha phosphatidylcholine a été préparée à partir de lécithine de jaune d'oeuf (pureté : 99 %, masse molaire : 789) ou de soja. L'amphotéricine B était celle de grade i.v. (pureté : 94%, masse molaire : 924). L'évaporation a été effectuée en utilisant un évaporateur du type Buchi evaporateur rotatif (sauf indication contraire).

### * Procédés de préparation

Toutes les préparations décrites ci-après contenaient de l'amphotéricine B à une concentration finale de 5 mg/ml.

### Procédure 1 : Préparation de suspension d'amphotéricine B

25 mg d'amphotéricine B on été dissous dans du méthanol (0,1 mg/ml). Le solvant a été évaporé et le résidu sec a été repris dans 5 ml d'une solution saline de tampon phosphate de pH 7,4 (PBS).L'échantillon a été soniqué.

### Procédure 2 :

Les préparations de microparticules d'Amphotéricine B ont été réalisées en utilisant un évaporateur à film ascendant de QUICK FIT.

Dans un erlen de 5 Litres, on dissout 1 gramme d'Amphotéricine B et 0,85 grammes de L-alpha-phosphatidylcholine (egg lécithine pureté supérieure à 99 %) dans 1,5 litres d'un mélange de chloroforme-méthanol (1:1 en volume), auxquels on rajoute, quand la solution est limpide, 100 ml de NaCl 0,9%.

Les solvants sont éliminés sous vide dans l'évaporateur à film ascendant. La température maximale de la solution, en haut de l'évaporateur est de 35°-45°C. Après séparation des phases, on récupère la phase aqueuse contenant les microparticules, que l'on soumet à un deuxième cycle d'évaporation.

Le système est rincé à l'aide d'eau distillée et le volume final est amené à 200 ml à l'aide d'un évaporateur rotatif, dont le bain chauffant ne dépasse pas 40°C.

On récupère généralement 82 à 86% de l'amphotéricine sous forme d'une suspension de microparticules à la concentration comprise entre 4 et 5 mg/ml.

### Procédure 3

25 mg d'amphotéricine B ont été dissous dans du méthanol (5 mg/ml) en présence d'un équivalent de NaOH (205 µl d'une solution méthanolique 0,132 M). 21,2 mg de phosphatidylcholine ont été introduite dans le mélange. Ceci correspond à un rapport molaire 1:1 entre l'amphotéricine B et la phosphatidylcholine. La préparation a été agitée jusqu'à complète dissolution. 5 ml de NaCl 0,9% en poids ont été ajoutés. La formation d'un précipité jaune est observée. Le mélange est ensuite neutralisé par addition d'un équivalent d'acide (30,7 µl d'une solution 0,88 M HCl dans le méthanol). Le méthanol a ensuite été évaporé et le volume final a été ajusté à 5 ml avec une solution de NaCl 0,9 % en poids.

### Procédure 4

21,2 mg de phosphatidylcholine ont été déposés sous forme de film au fond d'un ballon de 500 ml par rotoévaporation du chloroforme d'une solution chloroformique du phospholipide. Le film a été solubilisé dans 250 ml d'une solution d'amphotéricine B dans le méthanol (25 mg à 0,1 mg/ml) et à nouveau évaporé à l'évaporateur rotatif, jusqu'à obtention d'un film fin. Après addition de 5 ml de solution PBS, le mélange a été soniqué pendant 0,5 heures.

### Procédure 5

Le même procédé que la "procédure 3" a été suivi excepté le fait que le phospholipide était dissous dans le chloroforme avant l'addition de la solution d'amphotéricine B. Dans ce cas, aucun précipité n'a été observé après addition de la solution aqueuse PBS (2 ml). Toutefois un solide jaune précipite durant l'évaporation des solvants.

### Procédure 6

Un procédé identique à celui de la "procédure 4" a été suivi en utilisant toutefois de la lécithine de soja à la place de la lécithine de jaune d'oeuf.

### Procédure 7

Une procédure identique à la "procédure 3" a été suivie en utilisant de la lécithine de soja hydrogénée à la place de la lécithine de jaune d'oeuf.

### Procédure 8

Une procédure identique à la "procédure 3" à été suivie en utilisant de l'eau à la place d'une solution de NaCl 0,9 %.

### Procédure 9

Une procédure identique à la "procédure 3" a été suivie en utilisant un solution de lactose 5 % à la place de la solution de NaCl 0,9 %.

### Procédure 10

Une procédure identique à la "procédure 3" a été suivie excepté le fait que seulement 1 ml d'une solution de NaCl 0,9 % a été ajouté à la solution. Dans ce cas, la précipitation n'était pas complète lors de l'apporte de la solution aqueuse. Cette précipitation était parachevée lors de la phase de neutralisation.

### Procédure 11

Même procédure que la "procédure 3" excepté que le mélange a été neutralisé par addition d'un équivalent de HCl en solution méthanolique avant l'addition de la solution de NaCl 0,9 %.

### Procédure 12

25 mg d'amphotéricine B ont été dissous en présence d'un équivalent de HCl dans une solution méthanolique à une concentration de 2 mg/ml dans le but d'obtenir une solution à 5 mg/ml de médicament sans trop de dégradation. Le solvant a été évaporé et le composé dissous dans le volume requis de méthanol. 21,2 mg de phosphatidylcholine de jaune d'oeuf ont été introduits dans le mélange. La préparation a été agitée jusqu'à complète dissolution. 5 ml de solution de NaCl 0,9 % ont été ajoutés. La formation d'un précipité jaune a été observée. Le mélange a été neutralisé par addition d'un équivalent d'acide. Le méthanol a été évaporé et le volume final ajusté à 5 ml avec une solution de NaCl 0,9 %.

### Procédure 13

Même procédure que la "procédure 12" excepté que le mélange a été neutralisé par addition d'un équivalent de NaOH dissous dans du méthanol avant l'addition de la solution de NaCl 0,9 %.

### Procédure 14

Même procédure que la "procédure 13", excepté que le phospholipide a été dissous dans le chloroforme avant l'addition de la solution d'amphotéricine B. Aucun précipité n'a été observé après l'addition de NaCl 0,9 % (2 ml). Un précipité jaune apparaît durant l'évaporation des solvants.

### Procédure 15

Même procédure que pour la "procédure 3" excepté que la lécithine est remplacée par un mélange de 1,2 - dimiristoyl - sn - glycerol (3) phosphocholine (12,84 mg) et de 1,2 - dimiristoyl - sn - glycero (3) phospho (1) - rac - glycérol (5.59 mg).

### Procédure 16

2 g d'amphotéricine B en suspension dans 500 ml de méthanol sont dissous par addition d'un équivalent de NaOH en solution dans le méthanol. 1.7 g de lécithine d'oeuf (1 équivalent) sont ajoutés à la solution limpide. Cette dernière est agitée jusqu'à dissolution complète du lipide. Sous agitation énergique, l'addition de 300 à 500 ml d'eau suivie de I'ajustement du pH de la solution à pH 7,8 entraine la précipitation des microparticules. Le méthanol est évaporé sous vide en utilisant un évaporateur à film ascendant (Quick Fit). La température maximale au haut de la colonne d'évaporation est de 35° à 45°C.

### * Méthodes

### Centrifugation en gradient de densité

(A.S. Janoff, L.T. Boni, M.C. Popescu, S.R. Minchey, P.R. Cullis, T.D. Madden, T. Taraschi, S.M. Gruner, E. Shyamsunder, M.W. Tate, R. Mendelsohn, and D. Bonner, Proc. Nat. Acad. Sci USA 85, 6122-6126 (1988)
500 µl d'un échantillon ont été déposés sur un gradient de sucrose continu (d = 1.0 à 1.18 g/ml) dans une solution de NaCl 150 mM/Hepes 20 mM, pH 7,4. Le gradient a été centrifugé pendant 21 heures à 22°C dans un rotor SW-41 (Beckman) à 230 000 x g. Après centrifugation, le gradient est fractionné en fraction de 0,53 ml et dosé quant au continu d'amphotéricine B et de phospholipide.

### Dosage du phospholipide

(M. Takayama, S. Itom, T. Nagasaki, I. Tanimizu, Clinica Chimica Acta, 79 (1977) 93-98 "A new Enzymatic method for determination of serum choline-containing phospholipids")
Classiquement, 20 µl d'un échantillon ont été mélangés avec 3 ml d'une solution d'enzyme (Boehringer Mannheim Gmbh, kit 691844) et incubés à 37°C pendant 10 minutes. L'absorbance de l'échantillon a été mesurée à 500 nm. La solution de chlorure de choline (correspondant à 3 mg de PC/ml) a été utilisée comme témoin.

### 4.3 Résultats

### 4.3.1 Détermination de phospholipide dans les microparticules

Des microparticules ont été préparées à partir de 4,15 mg de lécithine d'oeuf et 5 mg d'amphotéricine B. Les microparticules ont été mises en suspension dans une solution de Doc 1 % et ont été dosées quant à leur contenu total en phospholipide.

Plusieurs dilutions de microparticules ont été préparées dans une solution de Doc 1 % pH 11,3 et dosées quant à leur contenu en phospholipide. Le dosage est précis à partir de 0,1 mg de phospholipide/ml jusqu'à au moins 5 mg/ml.

### 4.3.2 Précision des profils de densité

Un gradient de saccharose de 0 à 41 % en poids de sucrose a été obtenu en utilisant un générateur de gradient LKB (volume total : 11 ml). Le tube a été fractionné en aliquots de 0,53 ml et la densité des différentes fractions a été déterminée par gravimétrie. Il apparaît un gradient linéaire qui s'étale de 1 à 1,18 g/ml. Une expérience de contrôle a montré que le profil de densité est le même après centrifugation de 230 000 x g.

### 4.3.3 Dosage de phospholipide et l'amphotéricine B dans les microparticules obtenues selon les procédures 1 à 5

Comme il apparait au tableau 3 ci-après, l'amphotéricine et le phospholipide sont récupérés à peu près complètement quel que soit le procédé suivi.

**Tableau 3**

| Echantillon | Phospholipide mg/ml | %* | Amphotéricine mg/ml | %* |
|---|---|---|---|---|
| 1 | 0,00 | --- | 4,15 | 83,0 |
| 2 | 3,99 | n.d.** | n.d. | n.d. |
| 3 | 4,64 | 109,0 | 4,74 | 94,8 |
| 4 | 4,24 | 99,8 | 4,46 | 89,2 |
| 5 | 5, 05 | 118,8 | 5,06 | 101,2 |

| | | | | |
|---|---|---|---|---|
| * exprimé en pourcentage de la valeur théorique | | | | |
| ** valeur théorique non disponible | | | | |

### 4.3.4 Profil de densité des centrigugations en gradient de sucrose

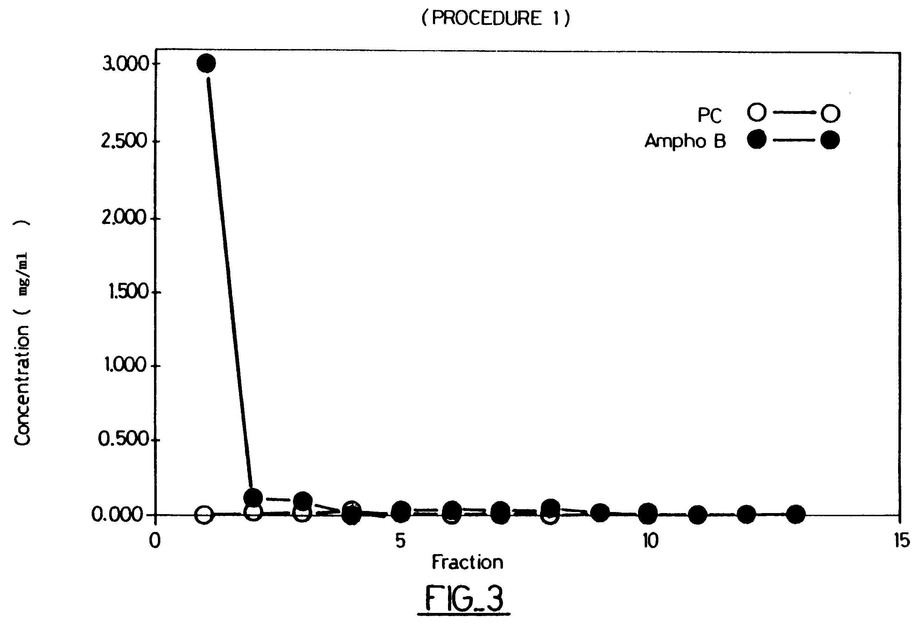

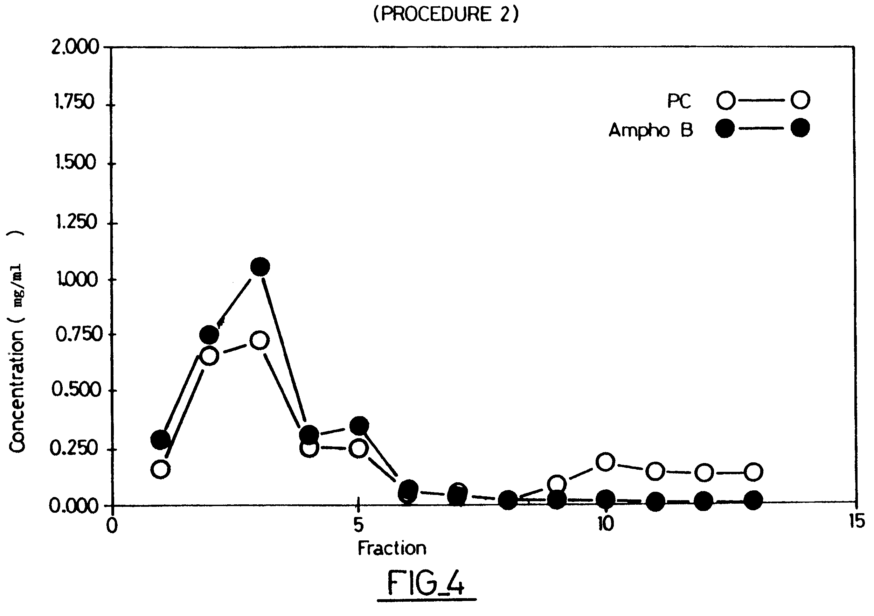

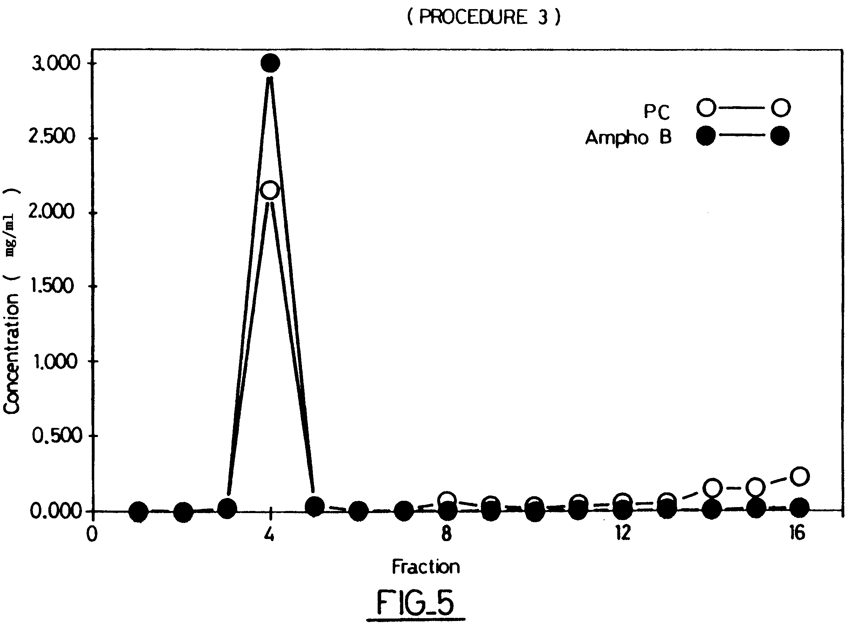

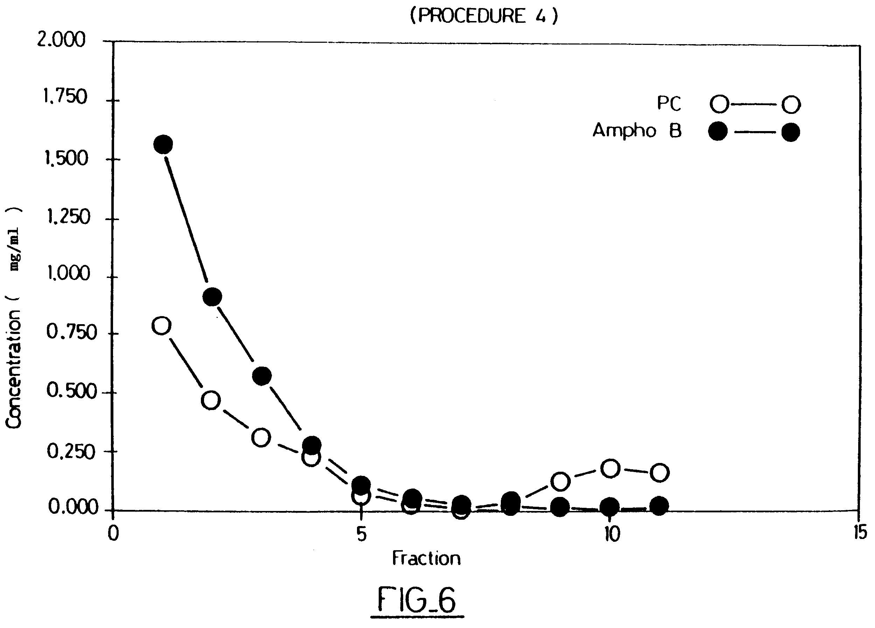

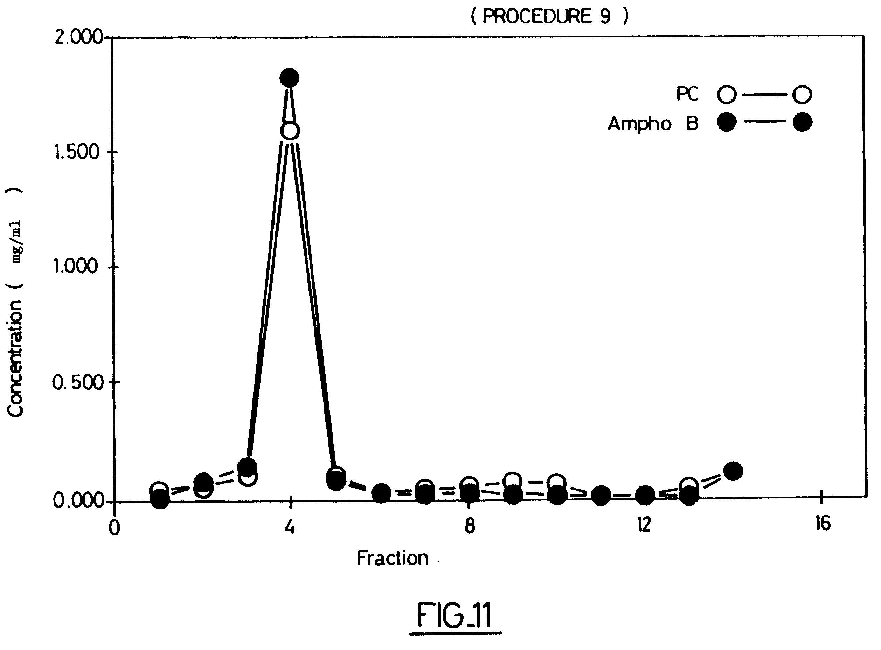

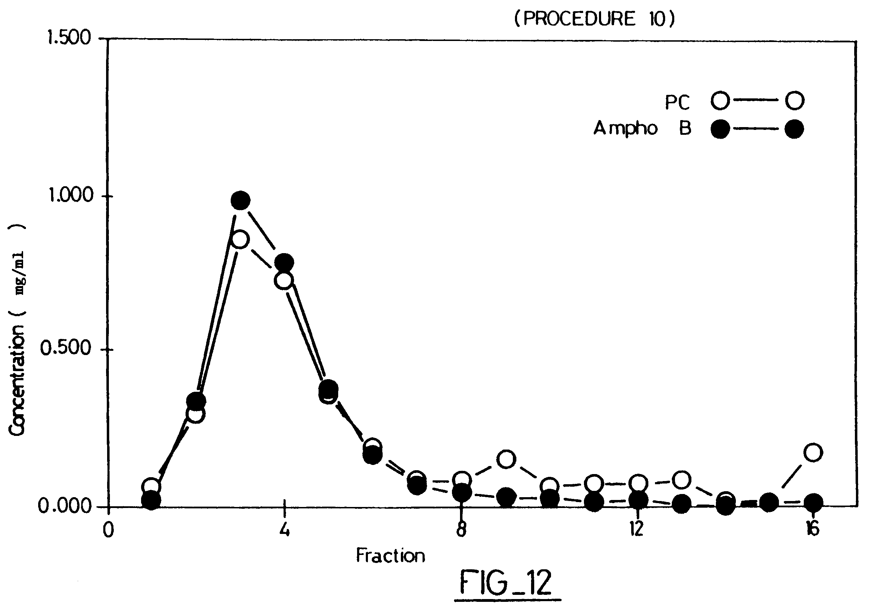

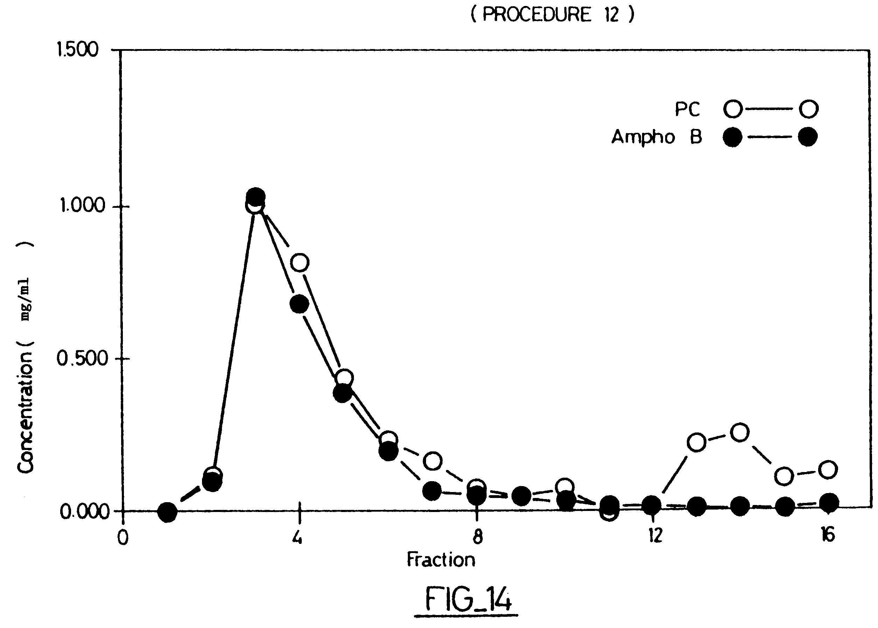

Les figures 3 à 16 montrent des profils de densité de sucrose isopycniques de 14 préparations d'amphotéricine B. Les microparticules obtenues dans les procédure 3 et 10 (figures 5 et 12 respectivement) donnent lieu à des profils de densité dans lesquels l'amphotéricine B est étroitement associé à la phosphatidylcholine dans une unique bande (1,13-1,15 g/ml). Les couches sont minces et sont nettement distantes du fond du tube. Le rapport molaire amphotéricine B/phospholipide et la densité de ces bandes uniques suggèrent que l'on a bien à faire à un complexe d'amphotéricine B/lipide. Les largeurs de couches de microparticules et les rapports molaires amphotéricine B/phospholipides observés pour ces différentes procédures sont reportés au tableau 4 ci-après.

Dans la procédure 1 (figure 3), l'amphotéricine a migré au fond du tube. Ce résultat indique que la centrifugation de gradient de densité est utile pour distinguer des microparticules d'amphotéricine B/phospholipide d'agrégats d'amphotéricine B libre.

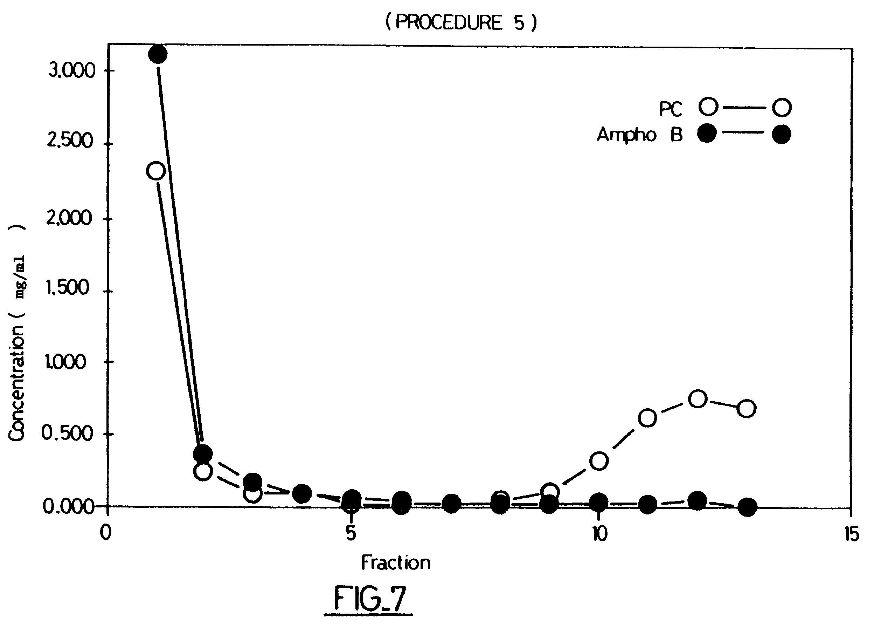

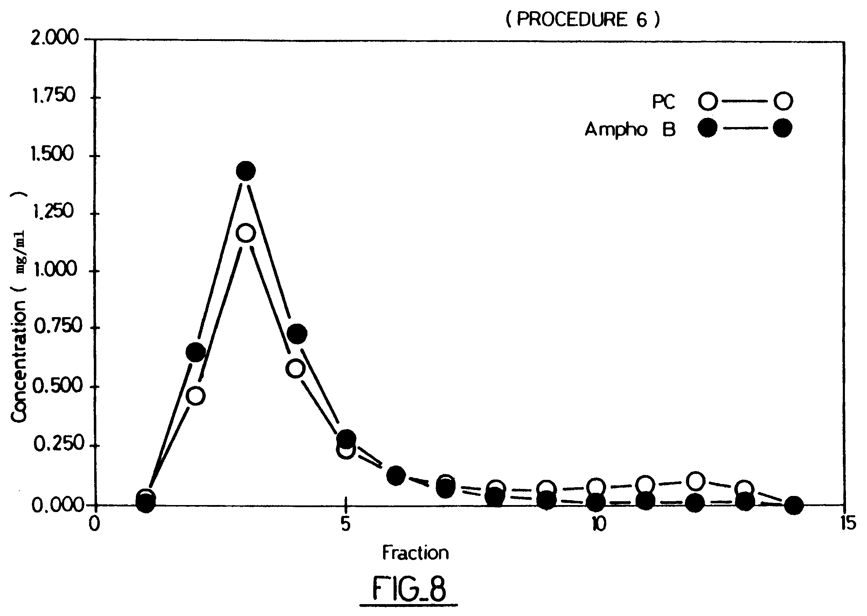

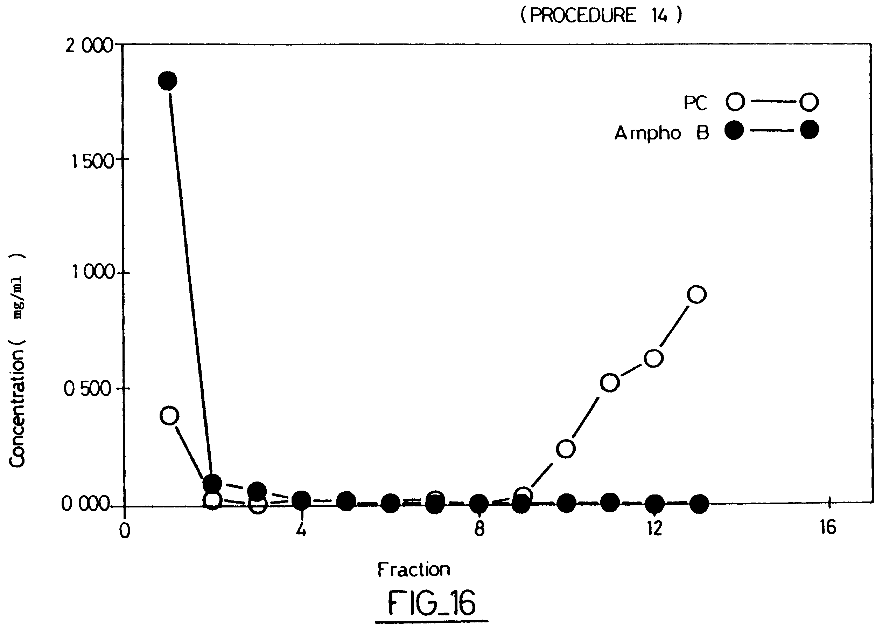

Les microparticules obtenues selon la procédure 5 (figure 7), la procédure 6 (figure 8) et la procédure 14 (figure 16) ont montré une distribution de densité bimodale très proche. La grande majorité de l'amphotéricine B était située au fond du tube avec une partie du lipide. Les phospholipides restants flottaient sous forme de couche opalescente Pour ces échantillons, il est difficile de démontrer la présence de microparticules.

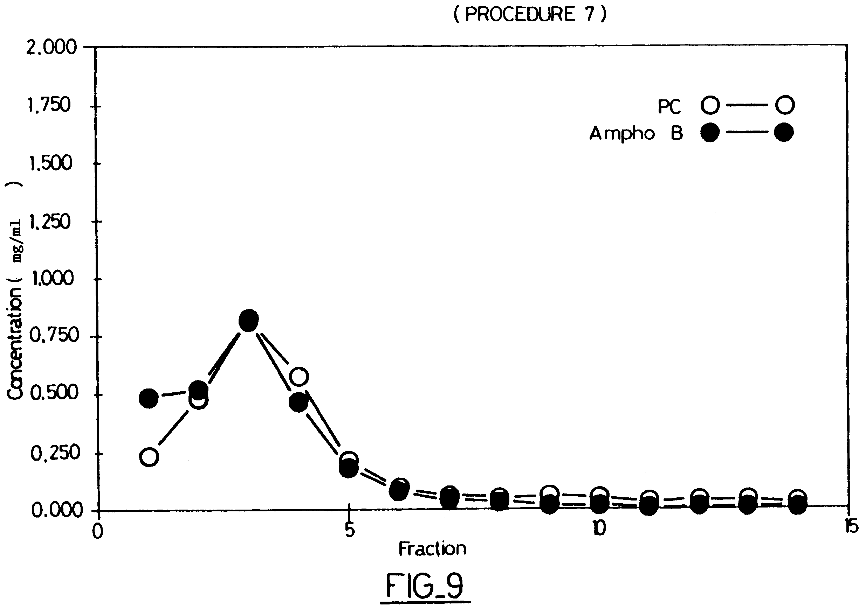

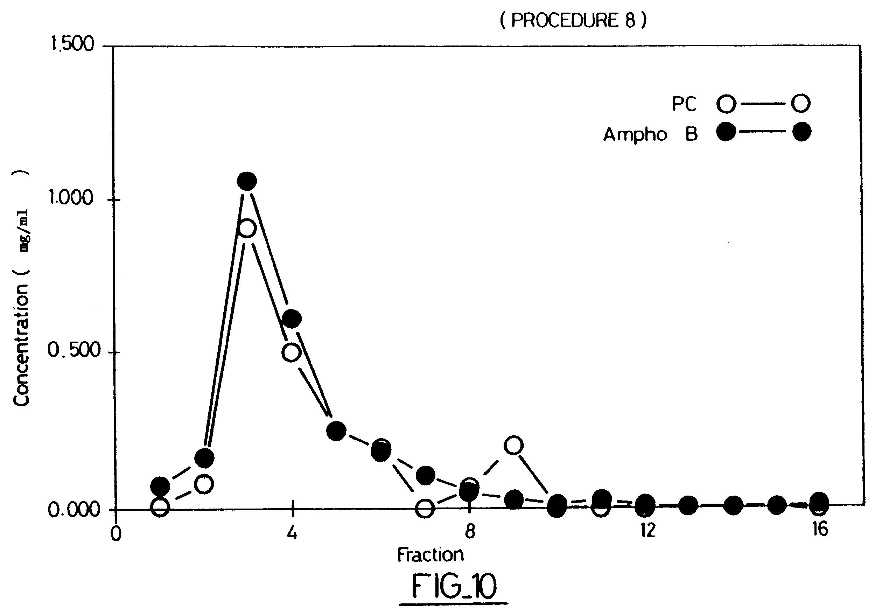

Les résultats obtenus avec des lécithines de soja et de soja hydrogénés (procédure 7, figure 9 et procédure 8, figure 10 respectivement) sont très proches de ceux observés avec les microparticules obtenues selon les procédures 3 et 10, mais les microparticules sont légèrement moins homogènes.

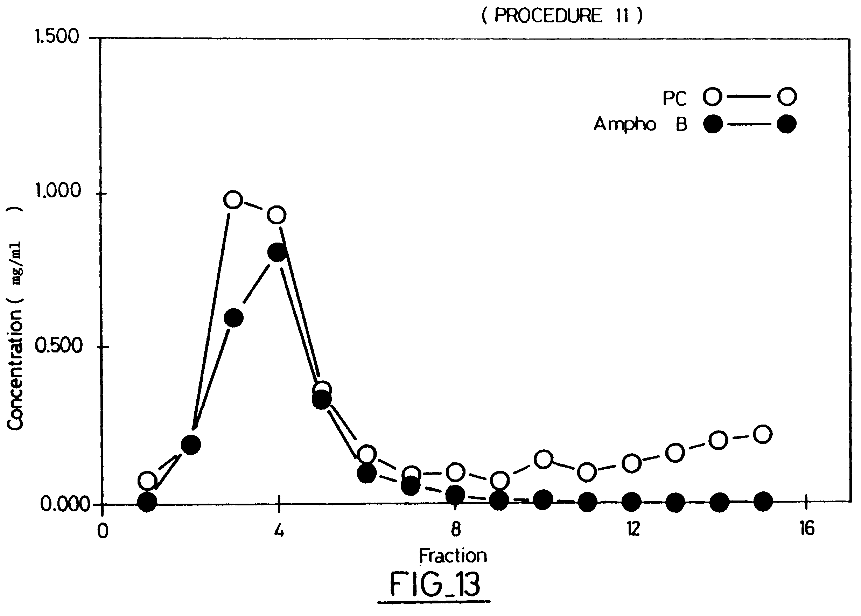

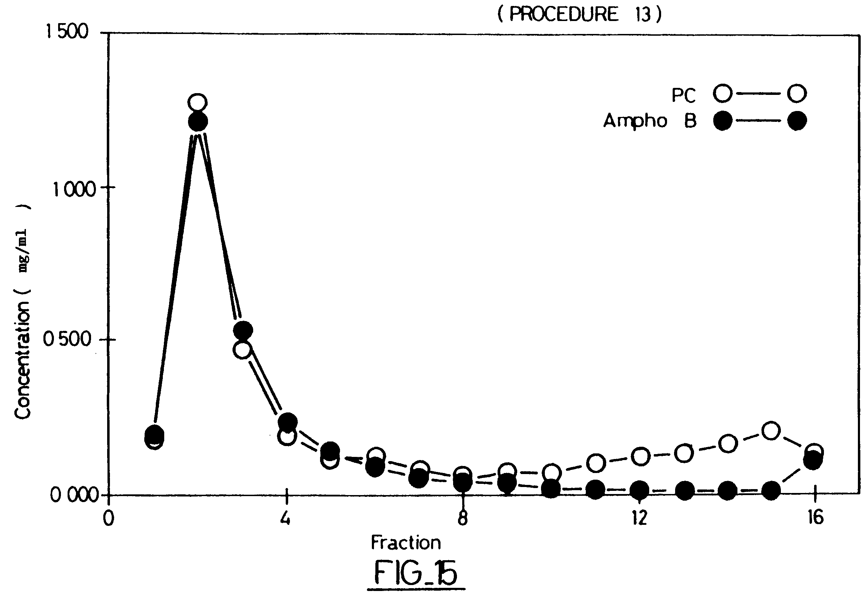

Les microparticules ont été obtenues quand on a ajouté une quantité inférieure de NaCl 0,9% (procédure 11, figure 13), quand la neutralisation intervenait avant l'addition de la solution aqueuse NaCl 0,9% (procédure 13, figure 15) ou quand l'amphotéricine B était dissoute dans un milieu acide (procédure 14, figure 16).

**Tableau 4**

| Procédure | Largeur de couche (cm) | Rapport molaire AmphoB/phospholipide ** |
|---|---|---|
| 1 | --- | --- |
| 2 | 0,4 | 1,37 |
| 3 | 0,1 | 1,15 ± 0,04 (n = 4) |
| 4 | --- | --- |
| 5 | --- | --- |
| 6 | 0,6* | 1,04 |
| 7 | 0,4* | 0,87 |
| 8 | 0,8 | 0,99 |
| 9 | 0,1 | 0,97 |
| 10 | 1,3 | 0,97 |
| 11 | 0,8 | 0,74 |
| 12 | 0,4 | 0,87 |
| 13 | 0,3 | 0,82 |
| 14 | --- | --- |

| | | |
|---|---|---|
| * mesuré après lyophilisation | | |
| ** rapport calculé pour la fraction de gradient contenant la quantité la plus important de microparticules | | |

### 5. Lyophilisation

Les microparticules sont conservées sous forme lyophilisée. la reconstitution après lyophilisation de la suspension de microparticules en solution auqueuse conduit à des particules présentant les mêmes caractéristiques physicochimiques et biologiques qu'avant la lyophilisation.

Afin de conserver l'intégrité des microparticules, la lyophilisation doit se faire en présence de divers excipients tels que le glucose, le lactose, tampon phosphate, tris, albumine, carboxyméthylcellulose.

### 6. Conclusions

Les préparations les plus homogènes ont été obtenues suivant les procédures 3 et 10, c'est-à-dire dissolution de l'amphotéricine B à une concentration de 5 mg/ml en présence d'un équivalent de base, utilisation de phosphatidylcholine de jaune d'oeuf et addition de solution de NaCl 0,9 % ou de lactose 5% (1 ml pour 5 mg d'amphotéricine B) avant neutralisation du mélange. La préparation à base de lécithine de soja ou de lécithine de soja hydrogéné était légèrement moins homogène.

Des microparticules ont aussi été préparées par neutralisation du mélange avant l'addition de la solution NaCl 0,9 % ou par dissolution de l'amphotéricine dans le méthanol en utilisant un équivalent de HCl. Selon cette dernière procédure, le mélange a été neutralisé avant ou après l'addition de la solution NaCl 0,9%. Dans toutes ces préparations, les microparticules étaient moins homogènes comme le montre la centrifugation en gradient de sucrose.

Aucune microparticule n'a été obtenue quand du chloroforme était utilisé pour préformer un film de lécithine de jaune d'oeuf ou pour dissoudre le phospholipide avant l'addition de la solution méthanolique basique d'amphotéricine B.

### EXEMPLE 2: Microparticules préparées avec d'autres solvants

### A) Utilisation du DMF

50 mg d'amphotéricine B sont dissous à 0°C dans 1 ml de diméthylformamide en présence d'un équivalent d'HCl dans le méthanol (47 µl d'une solution 0,87 M) 42,5 mg (1 équivalent) de phosphatidylcholine sont introduits dans la solution. La préparation est agitée jusqu'à complète dissolution, l'addition d'1 ml d'eau entraîné la précipitation des microparticules. La solution est neutralisée par addition d'1 équivalent de NaOH. La diméthylformamide est éliminée en majorité par trois cycles de centrifugation-élimination du surnageant et resuspension en solution aqueuse.

### B) Utilisation du propane diol 1.2

1 g d'amphotéricine B est dissous dans 250 ml de propane diol 1.2 (4 mg/ml) en présence de 1 équivalent de KOH (10 ml d'une solution à 0,1 M de KOH dans l'éthanol). 850 mg de phosphatidylcholine en solution dans 3 ml d'éthanol sont introduite dans la solution ceci correspond à un rapport molaire 1:1 entre l'amphotéricine B et la phosphatidylcholine. La solution est agitée à 800 tours/minute à température ambiante et neutralisée à l'aide de 10 ml de HCl 0,1 M (1 équivalent). 250 ml de H₂O sont ajoutés au mélange entraînant la précipitation des microparticules. L'élimination du propane diol 1.2 a été conduite par filtration tangentielle.

### C) Utilisation du diméthylacétamide (DMA)

La solubilité de l'amphotéricine B dans le DMA (6 mg/ml) est suffisante pour éviter l'utilisation d'une base ou d'un acide.

300 mg d'amphotéricine B sont dissous dans 50 ml de DMA (6 mg/ml), 255 mg de lécithine d'oeuf dans l'éthanol (1 ml) sont ajoutés à la solution. Le mélange est agité à 1000 tours/minute et 75 ml d'eau sont ajoutés au mélange entraînant la précipitation des microparticules. Le DMA est éliminé par centrifugation vu l'incompatibilité des membranes de filtration avec le DMA.

### Centrifugation en gradient de sucrose

La centrifugation sur gradient de sucrose montre pour les préparations obtenues dans la DMF, le propane diol 1.2 et le DMA, une bande située à mi-hauteur du tube et caractérisée par un rapport molaire amphotéricine B/phospholipide compris entre 0,8 et 1,4.

Aucune trace d'amphotéricine B libre ou phospholipide libre n'est observée.

### EXEMPLE 3: Microparticules d'amphotéricine B et de divers mélanges de phospholipides.

### EXEMPLE 3-1

1 gr (1,1 mmole) d'Amphotericine-B a été dissous dans 250 ml de méthanol refroidi dans un bain de glace en présence d'un équivalent de NaOH. Un mélange de 0,367g. (0.5 équivalent) de DMPO et 0,373g. (0,5 équivalent) de DMPG en solution dans 80 ml de méthanol a été ajouté à la solution d'Amphotéricine-B refroidie dans un bain de glace. Le rapport molaire Amphotéricine-B phospholipides est de 1 : 1. Les microparticules ont été obtenues par précipitation après addition de 300 ml de tampon phosphate 50mM pH 7,8, 6% lactose et neutralisation du mélange. Le mélange est chauffé durant 30 minutes à 60°C et le méthanol est éliminé par évaporation sur film ascendant.

### EXEMPLE 3-2

Même procédé que celui décrit dans l'exemple 1 mais en partant de 0,35 équivalent de DMPC et 0,15 équivalent de DMPG calculésl par rapport à l'Amphotéricine-B. Le rapport molaire Amphotéricine-B-phospholipides est donc de 2:1.

### EXEMPLE 3-3

Même procédé que celui décrit dans l'exemple 1 mais en partant de 0,75 équivalent de DMPC et 0,08 équivalent de DMPG calcules par rapport à l'Amphotéricine-B. Ce qui correspond à un rapport molaire Amphotéricine-B-phospholipides de 1,2 : 1.

### EXEMPLE 3-4

Même procédé que celui décrit dans l'exemple 1 mais en partant de 0.7 équivalent de lécithine d'oeuf et 0,3 équivalent de DMPG calculé par rapport à l'Amphotéricineb-B. Ce qui correspond à un rapport molaire Amphotéricine-B-phospholipides de 1 : 1.

### EXEMPLE 3-5

Même procédé que celui décrit dans l'exemple 1 mais en partant de 0,7 équivalent de lécithine de soja hydrogénée et 0,3 équivalent de DMPG calculé par rapport à l'Amphotéricine-B. Ce qui correspond à un rapport molaire Amphotéricine-B-phospholipides de 1 : 1.

### EXEMPLE 3-6

1,85 g (2 mmoles) d'Amphotéricine-B ont été dissous dans 500 ml de propane diol 1,2 en présence d'une équivalent de NaOH (200 µl d'une solution NaOH 10N). Une solution contenant 0,45 g (0,33 équivalent) de DMPC et 0,46 g (0,33 équivalent) de DMPG en solution dans 100 ml de propane diol-1,2 a été ajouté à la solution d'Amphotéricine-B. La mise en solution du DMPG dans le propane diol-1,2 se fait par chauffage de la solution à 60°. Le rapport molaire Amphotéricine-B-phospholipides est de 1,5 : 1.

Les microparticules ont été obtenues par précipitation après addition d'un litre de tampon phosphate 50mM pH 7,8, 6% lactose et neutralisation du mélange (addition de 2 ml d'une solution HCl 1 N).

Après chauffage du mélange durant 30 minutes à 60° C, le propane diol-1,2 est éliminé par filtration tangentielle.

### EXEMPLE 3-7

Même procédé que celui décrit dans l'exemple 6 mais en partant d'un rapport molaire Amphotéricine-B-phospholipides de 2 : 1.

### EXEMPLE 3-8

Même procédé que celui décrit dans l'exemple 6 mais en partant d'un rapport molaire Amphotéricine-B-phospholipides de 1 : 1.

### EXEMPLE 3-9

Même procédé que celui décrit dans l'exemple 6 mais en partant de 0.35 équivalent de DMPC et 0.15 équivalent de DPPS. Ce qui correspond à un rapport molaire Amphotéricine-B-phospholipides de 2 : 1.

### EXEMPLE 3-10

1,85 g (2 mmoles) d'Amphotéricine-B ont été dissous dans 400 ml d'éthanol refroidi dans un bain de glace en présence d'un équivalent de HCl (2 ml d'une solution HCl 1 N). Une solution contenant 0,45 g (0,33 équivalent) de DMPC et 0,46 g (0,33 équivalent) de DMPG en solution dans 100 ml d'éthanol contenant 2 ml de HCl 1 N a été ajouté à la solution d'Amphotéricine-B refroidie dans un bain de glace. Le rapport molaire Amphotéricine-B-phospholipides est de 1,5 : 1. Les microparticules ont été obtenues par précipitation après addition sous agitation d'un titre d'eau et neutralisation du mélange (addition de 4 ml d'une solution NaOH 1 N). Après chauffage du mélange durant 30 Min à 60° C, l'éthanol est éliminé par filtration tangentielle ou par évaporation sur film ascendant.

### EXEMPLE 3-11

1,85 g (2 mmoles) d'Amphotéricine-B ont été dissous dans 400 ml d'éthanol refroidi dans un bain de glace en présence d'un équivalent de HCl (2 ml d'une solution HCl 1 N). Une solution de 2,85 g (3 équivalents) de valerate de cholestérol et 0,689 g (0,3 équivalent) de DMPG dans de l'éthanol contenant 2 ml de HCl 1 N est ajouté à la solution d'Amphotéricine-B refroidie dans la glace. Le rapport molaire Amphotéricine-B-lécithine est de 0,3 : 1. Le mélange est agité énergiquement et les microparticules sont obtenues par précipitation après addition d'un titre d'eau et neutralisation du mélange (addition de 4 ml de NaOH 1N. Après chauffage du mélange durant 30 minutes à 60° C, l'éthanol est éliminé par filtration tangentielle ou sur film ascendant.

Ces préparations des exemples 3-1 à 3-11 sont homogènes, montrent une absence d'effet hémolytique, une activité in-vitro sur candida tropicalis égale ou légèrement supérieure à celle de la fungizone et une diminution de la toxicité mesurée in-vivo sur des souris OF1 en comparaison à celle de la Fungizone. On observe dans certains cas une LD_{50 supérieure à 30.}

L'utilisation de DSPC, DSPG et DPPS conduit à des préparations très homogènes de microparticules présentant une bonne activité in-vitro sur candida tropicalis (ED₅₀ égale ou légèrement meilleure que la Fungizone) et montrant même une diminution de la toxicité aiguë déterminée in-vivo sur souris OF1 et comparée à l'Amphotéricine-B utilisée sous forme de Fungizone.

**TABLEAU 5**

| LD₅₀ des préparations correspondants aux exemples 1 à 11 Etudes conduites sur souris OF1 | |
|---|---|
| EXEMPLE | LD₅₀ |
| 1 | 28,06 |
| 2 | >30 |
| 3 | 27,06 |
| 4 | >30 |
| 5 | >30 |
| 6 | 29,5 |
| 7 | 21,7 |
| 8 | 29,5 |
| 9 | 19 |
| 10 | >30 |
| 11 | >30 |

## Revendications

1. Procédé de préparation de microparticules lipidiques d'aspect microcristallin d'une substance insoluble dans l'eau présentant une affinité pour les phospholipides, et d'au moins un phospholipide, microparticules stables en suspension dans une solution aqueuse, caractérisé en ce que :
a) on dissout ladite substance et le ou lesdits phospholipides dans un solvant organique commun de ladite substance et du ou desdits phospholipides en milieu basique ou acide ;
b) on mélange la solution de ladite substance et du ou desdits phospholipides à une solution aqueuse dans une quantité telle qu'une insolubilisation sous forme de précipité se produit ;
c) on neutralise la solution par addition d'acide ou de base respectivement ;
d) on élimine le solvant pour récupérer une solution aqueuse contenant les microparticules sous forme de microsuspensions.

2. Procédé selon la revendication 1, caractérisé en ce que le rapport molaire entre le ou les phospholipides et ladite substance est inférieur à 2, de préférence compris entre 0,5 et 1.

3. Procédé selon l'une des revendications précédentes, caractérisé en ce que ladite substance est choisie parmi les agents antimycotiques, macrolides polyènes.

4. Procédé selon la revendication précédente, caractérisé en ce que ladite substance est choisie parmi la nystatine, l'amphotéricine B et leurs dérivés antifongiques.

5. Procédé selon l'une des revendications 1 à 4, caractérisé en ce que le phospholipide est choisi parmi la phosphatidylcholine, la dimyristoylphosphatidylcholine (DMPC), la distéarylphosphatidylcholine (DSPC), la dipalmitoylphosphatidylcholine (DPPC), la phosphatidylétanolamine, la phosphatidylsérine, la dipalmitoylphosphatidyl serine (DPPS), la phosphatidylinositol, la phosphatidylglycérol, la dimyristoyl phosphatidyl glycérol (DMPG), la distearyl phosphatidylglycérol (DSPG), la 3'-O-lysylphosphatidylglycérol, la diphosphatidylglycérol, ou encore des esters de cholestérol, seuls ou en mélange.

6. Procédé selon l'une des revendications précédentes, caractérisé en ce que le phospholipide est de la phosphatidylcholine ou une dimyristoylphosphatidylcholine en mélange avec de la dimyristoylphosphatidylglycérol en rapport molaire 5:5 à 9:1.

7. Procédé selon l'une des revendications précédentes, caractérisé en ce que le solvant organique de la solution de phospholipide et de ladite substance est choisi parmi les solvants organiques tels que le méthanol, le DMF, le DMA, le propylène glycol ou l'éthanol.

8. Procédé selon l'une des revendications précédentes, caractérisé en ce que la solution aqueuse est de l'eau pure, une solution saline ou une solution de saccharide.

9. Procédé selon la revendication précédente, caractérisé en ce que la solution aqueuse est une solution de lactose de 1 à 10 % en poids.

10. Procédé selon l'une des revendications 8 ou 9, caractérisé en ce que la solution aqueuse est un tampon phosphate 50mM pH=7,8 contenant 6% de lactose.

11. Procédé selon l'une des revendications 1 à 10, caractérisé en ce que ladite substance est choisie parmi les agents antimycotiques, macrolides du type polyènes tels que l'amphotéricine B ou la nystatine et à l'étape a) est dissoute dans une solution méthanolique ou une solution de propylène glycol en milieu basique ou dans une solution de DMF ou d'éthanol en milieu acide, ou encore dans une solution de DMA en milieu neutre ou acide.

12. Procédé selon l'une des revendications 1 à 11, caractérisé en ce que à l'étape a), la quantité d'acide ou de base utilisée est de 1 à 1,5 équivalent d'acide ou de base par rapport à la quantité de substance active, et à l'étape c), on neutralise avec une même quantité de base ou d'acide respectivement.

13. Procédé selon l'une des revendications 1 à 12, caractérisé en ce que on chauffe le mélange avant l'étape d).

14. Préparation de microparticules pouvant être obtenue par le procédé selon l'une des revendications précédentes, caractérisée en ce que les microparticules sont de tailles homogènes.

## Claims

1. Process for preparing lipid microparticles, of microcrystalline appearance, of a water-insoluble substance displaying an affinity for phospholipids and of at least one phospholipid, the microparticles being stable in suspension in an aqueous solution, characterized in that:
a) the said substance and the said phospholipid or phospholipids are dissolved in a common organic solvent for the said substance and for the said phospholipid or phospholipids in a basic or acidic medium;
b) the solution of the said substance and of the said phospholipid or phospholipids is mixed with an aqueous solution in an amount such that an insolubilization in the form of a precipitate takes place;
c) the solution is neutralized by adding acid or base, respectively;
d) the solvent is removed to recover an aqueous solution containing the microparticles in the form of microsuspensions.

2. Process according to Claim 1, characterized in that the mole ratio of the phospholipid or phospholipids to the said substance is less than 2, and preferably between 0.5 and 1.

3. Process according to one of the preceding claims, characterized in that the said substance is chosen from antimycotic agents and polyene macrolides.

4. Process according to the preceding claim, characterized in that the said substance is chosen from nystatin, amphotericin B and their antifungal derivatives.

5. Process according to one of Claims 1 to 4, characterized in that the phospholipid is chosen from phosphatidylcholine, dimyristoylphosphatidylcholine (DMPC), distearylphosphatidylcholine (DSPC), dipalmitoylphosphatidylcholine (DPPC), phosphatidylethanolamine, phosphatidylserine, dipalmitoylphosphatidylserine (DPPS), phosphatidylinositol, phosphatidylglycerol, dimyristoylphosphatidyl glycerol (DMPG), distearyl phosphatidylglycerol (DSPG), 3'-O-lysylphosphatidylglycerol, diphosphatidylglycerol or alternatively cholesterol esters, alone or mixed.

6. Process according to one of the preceding claims, characterized in that the phospholipid is phosphatidylcholine or a dimyristoylphosphatidylcholine mixed with dimyristoylphosphatidylglycerol in a 5:5 to 9:1 mole ratio.

7. Process according to one of the preceding claims, characterized in that the organic solvent of the solution of phospholipid and of the said substance is chosen from organic solvents such as methanol, DMF, DMA, propylene glycol or ethanol.

8. Process according to one of the preceding claims, characterized in that the aqueous solution is pure water, a saline solution or a solution of saccharide.

9. Process according to the preceding claim, characterized in that the aqueous solution is a lactose solution containing 1 to 10 % by weight.

10. Process according to either of Claims 8 and 9, characterized in that the aqueous solution is a 50mM phosphate buffer, pH 7.8, containing 6 % of lactose.

11. Process according to one of Claims 1 to 10, characterized in that the said substance is chosen from antimycotic agents and macrolides of the polyene type such as amphotericin B or nystatin and, in step a), is dissolved in a methanolic solution or a solution of propylene glycol in a basic medium or in a solution of DMF or ethanol in an acidic medium, or alternatively in a solution of DMA in a neutral or acidic medium.

12. Process according to one of Claims 1 to 11, characterized in that, in step a), the amount of acid or base used is from 1 to 1.5 equivalents of acid or base relative to the amount of active substance, and, in step c), neutralization is performed with the same amount of base or acid, respectively.

13. Process according to one of Claims 1 to 12, characterized in that the mixture is heated before step d).

14. Preparation of microparticles which is capable of being obtained by the process according to one of the preceding claims, characterized in that the microparticles are of homogeneous sizes.

## Patentansprüche

1. Verfahren zur Herstellung von Lipid-Mikroteilchen mit mikrokristallinem Aussehen einer in Wasser unlöslichen Substanz, die eine Affinität für die Phospholipide aufweist, und mindestens eines Phospholipids, wobei die Mikroteilchen in Suspension in einer wäßrigen Lösung stabil sind, dadurch gekennzeichnet, daß man
a) die genannte Substanz und das oder die genannten Phospholipide in einem für die genannte Substanz und das oder die genannten Phospholipide gemeinsamen organischen Lösungsmittel in einem basischen oder sauren Milieu löst;
b) die Lösung der genannten Substanz und des oder der genannten Phospholipide mit einer wäßrigen Lösung in einer solchen Menge mischt, daß eine Insolubilisierung in Form eines Niederschlags auftritt;
c) die Lösung durch Zugabe einer Säure bzw. einer Base neutralisiert; und
d) das Lösungsmittel eliminiert zur Gewinnung einer wäßrigen Lösung, welche die Mikroteilchen in Form von Mikrosuspensionen enthält.

2. Verfahren nach Anspruch 1, dadurch gekennzeichnet, daß das Molverhältnis zwischen dem oder den Phospholipiden und der genannten Substanz unter 2, vorzugsweise zwischen 0,5 und 1, liegt.

3. Verfahren nach einem der vorhergehenden Ansprüche, dadurch gekennzeichnet, daß die genannte Substanz ausgewählt wird aus antimykotischen Agentien und Polyen-Makroliden.

4. Verfahren nach dem vorhergehenden Anspruch, dadurch gekennzeichnet, daß die genannte Substanz ausgewählt wird aus Nystatin, Amphotericin B und ihren Antifungi-Derivaten.

5. Verfahren nach einem der Ansprüche 1 bis 4, dadurch gekennzeichnet, daß das Phospholipid ausgewählt wird aus Phosphatidylcholin, Dimyristoylphosphatidylcholin (DMPC), Distearylphosphatidylcholin (DSPC), Dipalmitoylphosphatidylcholin (DPPC), Phosphatidylethanolamin, Phosphatidylserin, Dipalmitoylphosphatidylserin (DPPS), Phosphatidylinosit, Phosphatidylglycerin, Dimyristoylphosphatidylglycerin (DMPG), Distearylphosphatidylglycerin (DSPG), 3'-O-Lysylphosphatidylglycerin, Diphosphatidylglycerin oder auch den Cholesterinestern, einzeln oder in Form einer Mischung.

6. Verfahren nach einem der vorhergehenden Ansprüche, dadurch gekennzeichnet, daß es sich bei dem Phospholipid um das Phosphatidylcholin oder ein Dimyristoylphosphatidylcholin im Gemisch mit Dimyristoylphosphatidylglycerin in einem Molverhältnis von 5:5 bis 9:1 handelt.

7. Verfahren nach einem der vorhergehenden Ansprüche, dadurch gekennzeichnet, daß das organische Lösungsmittel der Lösung von Phospholipid und der genannten Substanz ausgewählt wird aus organischen Lösungsmitteln, wie Methanol, DMF, DMA, Propylenglycol oder Ethanol.

8. Verfahren nach einem der vorhergehenden Ansprüche, dadurch gekennzeichnet, daß es sich bei der wäßrigen Lösung um reines Wasser, eine Salzlösung oder eine Saccharidlösung handelt.

9. Verfahren nach dem vorhergehenden Anspruch, dadurch gekennzeichnet, daß es sich bei der wäßrigen Lösung um eine 1 bis 10 gew.-%ige Lactoselösung handelt.

10. Verfahren nach einem der Ansprüche 8 oder 9, dadurch gekennzeichnet, daß es sich bei der wäßrigen Lösung um einen 50 mM Phosphatpuffer, pH = 7,8, handelt, der 6 % Lactose enthält.

11. Verfahren nach einem der Ansprüche 1 bis 10, dadurch gekennzeichnet, daß die genannte Substanz ausgewählt wird aus den antimykotischen Agentien, den Makroliden vom Polyen-Typ wie Amphotericin B oder Nystatin und daß sie in der Stufe (a) in einer methanolischen Lösung oder in einer Propylenglycollösung in basischem Milieu oder in einer DMF-Lösung oder Ethanollösung in saurem Milieu oder auch in einer DMA-Lösung in neutralem oder saurem Milieu gelöst wird.

12. Verfahren nach einem der Ansprüche 1 bis 11, dadurch gekennzeichnet, daß in der Stufe (a) die verwendete Menge der Säure oder Base 1 bis 1,5 Äquivalente Säure oder Base, bezogen auf die Menge der aktiven Substanz, beträgt und daß man in der Stufe (c) mit einer gleichen Menge Base bzw. Säure neutralisiert.

13. Verfahren nach einem der Ansprüche 1 bis 12, dadurch gekennzeichnet, daß man die Mischung vor der Stufe (d) erwärmt.

14. Mikroteilchen-Präparat, das nach dem Verfahren nach einem der vorhergehenden Ansprüche erhältlich ist, dadurch gekennzeichnet, daß die Mikroteilchen homogene Abmessungen (Größen) haben.
